# EUROPÄISCHE PATENTSCHRIFT

(11) **EP 0 354 229 B1**
(45) Veröffentlichungstag und Bekanntmachung des Hinweises auf die Patenterteilung: **13.04.1994**
(21) Anmeldenummer: 89901281.9
(22) Anmeldetag: 17.01.1989
(51) Int. Cl.: A61B 5/02, G01L 11/00, G01N 21/41, H04R 23/00, A61B 17/22

(54) **SONDENHYDROPHON**
HYDROPHONIC PROBE
HYDROPHONE DE SONDAGE

(30) Priorität: 25.01.1988 DE 3802024
(43) Veröffentlichungstag der Anmeldung: 14.02.1990
(73) Patentinhaber: Eisenmenger, Wolfgang Prof. Dr., D-71634 Ludwigsburg (DE)
(72) Erfinder: EISENMENGER, Wolfgang, D-7140 Ludwigsburg (DE); STAUDENRAUS, Joachim, D-7303 Neuhausen (DE)
(74) Vertreter: Wilhelm & Dauster Patentanwälte European Patent Attorneys
(86) Internationale Anmeldenummer: EP8900053
(87) Internationale Veröffentlichungsnummer: WO8906512

(56) Entgegenhaltungen:
- DE-C- 3 302 089
- GB-A- 2 089 032
- US-A- 3 917 410
- US-A- 4 162 397
- US-A- 4 235 113
- US-A- 4 487 206
- US-A- 4 599 711
- US-A- 4 691 709

## Beschreibung

Sondenhydrophone dienen der Untersuchung und Charakterisierung von Wasserschallsignalen, z.B. Ultraschallimpulsen oder auch Stoßwellen nach ihrem raumzeitlichen Verlauf. Bekannt sind insbesondere piezoelektrische Sondenhydrophone unter Verwendung von kristallinen, keramischen oder polymeren Piezoelektrika, wobei das empfindliche Element Flächen- oder auch Kugelschalenform besitzt und sich am Ende einer in der Regel koaxialen elektrischen Zuleitung befindet. Eine Beschreibung der neueren Entwicklungen auf diesem Gebiet findet sich in der Zeitschrift Acustica 54, S. 23, 1983.

Das Ziel bei der Entwicklung von Sondenhydrophonen ist eine möglichst fehler- bzw. rückwirkungsfreie und empfindliche Wiedergabe der akustischen Signale mit höchster räumlicher und zeitlicher Auflösung. Hinzu kommt bei Stoßwellenuntersuchungen die Forderung nach hoher Lebensdauer im Druckbereich von 1 kbar, wie z.B. im Fokus von medizinischen Stoßwellengeräten sowie auch die Forderung nach einem möglichst großen Abstand zwischen der eigentlich empfindlichen Sondenspitze und dem anzuschließenden elektronischen Meßgerät, wie z.B. einem Verstärker. Letztere Forderung ergibt sich aus medizinischer Sicht bei der Bestimmung von Stoßwellendrucken im menschlichen Körper.

Bei den derzeit verfügbaren piezoelektrischen Sondenhydrophonen wird eine Bandbreite von 10 MHz bei einer Empfindlichkeit von ca. 1 mV/bar erreicht, wie in der Zeitschrift Acustica 54, S.23, 1983 und 64, S.85, 1987 beschrieben. Demgegenüber besitzen z.B. Stoßwellensignale Bandbreiten bis zu 1 GHz, vergl. Acustica 14, S.187, 1964.

Bei der Bandbreite von 10 MHz beträgt der effektive Sondendurchmesser etwa 0,1 mm. Da die Sondenspitze aus mehreren Schichten (Innenelektrode, Piezoelektrische Schicht, Außenelektrode) aufgebaut ist, stößt eine weitere Reduktion der Sondenabmessungen (zur Erhöhung der Bandbreite) auf erhebliche Schwierigkeiten. Außerdem nimmt die Sondenempfindlichkeit mit der Sondenoberfläche ab. Ebenso würde eine große Zuleitungslänge wegen der damit verbundenen Kabelkapazität einen weiteren Empfindlichkeitsverlust bewirken. Wegen des genannten Schichtaufbaus dieser Sonden ist ihre Haltbarkeit bei Stoßwellenuntersuchungen begrenzt. Im Druckbereich von 1 kbar und voll ausgebildeter Stoßfront liegt die Lebensdauer häufig unter hundert Stoßwellenexpositionen.

Aus der DE 33 02 089 C2 ist eine Vorrichtung zur Messung der optischen Brechzahl von Flüssigkeiten bekannt. Diese benützt einen Lichtwellenleiter, in welchem das eingespeiste Licht eine Vielzahl von Moden anregt und dessen Ende als angeschliffene und polierte Spitze mit im Querschnitt geradliniger Berandung ausgebildet ist. Die Anzahl der an dieser Spitze totalreflektierten Moden hängt von dem Brechungsindex der Flüssigkeit ab, in der die Spitze eingetaucht wird. Dieser Effekt wird zur statischen Messung des Brechungsindexes der Flüssigkeit ausgenutzt. Diese Vorrichtung eignet sich jedoch nicht zur Verwendung als Sondenhydrophon, wofür die Messung der Änderung des Brechungsindexes mit hoher zeitlicher Auflösung erforderlich ist. Zudem ist das zwangsläufig zugespitzte Sondenende bruchempfindlich und könnte den Druckstößen nicht dauerhaft standhalten, die maßgebliche Sondenfläche ist wegen der Anschrägung vergleichsweise groß, und es tritt ein die zeitliche Auflösung weiter hemmendes hohes Modenrauschen aufgrund der Modenvielzahl auf.

Aus der US-4 235 113 ist eine Vorrichtung zur Messung von Luftschall bekannt, bei der ein langer, spulenförmig gewickelter Lichtleiter sich in einem druckempfindlichen Medium befindet, welches von einer luftschallempfindlichen Membran begrenzt wird. Die Vorrichtung mißt die transmittierte Lichtintensität in Abhängigkeit von den auftreffenden Schallwellen. Sie eignet sich bereits wegen der räumlichen Ausdehnung des Sensorteils nicht für einen Einsatz als Sondenhydrophon zur Druckamplitudenmessung mit hoher zeitlicher und örtlicher Auflösung.

Aufgabe der Erfindung ist es daher, ein Sondenhydrophon mit möglichst einfachem Aufbau zu realisieren, dessen wirksame Dimensionen bei entsprechender Bandbreitensteigerung ohne Empfindlichkeitsverlust mit Durchmessern von weniger als 0,1 mm gewählt werden kann, das bei Stoßwellenexposition im 1 kbar-Bereich hohe Standzeiten besitzt und dessen Zuleitungslänge ohne Empfindlichkeitsverlust frei vorgegeben werden kann, wobei das Sondenhydrophon leicht kalibrierbar sein und hohe Reproduzierbarkeit besitzen soll.

Diese Aufgabe wird erfindungsgemäß durch ein Sondenhydrophon mit den Merkmalen des Anspruchs 1 gelöst.

Als Drucksonde wird statt eines piezoelektrischen Sondenhydrophons ein Lichtleiter in Form einer Glas- oder Polymerfaser in das akustische Wellenfeld eingeführt und die zeitliche Variation der Lichtreflexion an der Grenzfläche des Endes des Lichtleiters gegenüber der Flüssigkeit während der zeitlichen Druckänderung als Hydrophonsignal verwendet. Die Lichtreflexion an der Lichtleiterendfläche ist über den Brechungsindex - Dichte-Zusammenhang mit der Druckamplitude in der Flüssigkeit verknüpft. Bei Druckanstieg wird die Dichte und damit der Brechungsindex in der Flüssigkeit erhöht. Ein solcher Zusammenhang besteht grundsätzlich auch für das Lichtleitermaterial, wobei jedoch wegen der geringeren Kompressibilität des festen Lichtleitermaterials gegenüber der Flüssigkeit die druckbedingte Brechungsindexänderung der Flüssigkeit überwiegt. Die Änderung der Lichtreflexion wird hierbei über den zeitlichen Intensitätsverlauf des reflektierten Lichtes bei bekannter Einstrahlung photoelektrisch registriert. Weitere Ausgestaltungen sind in den Unteransprüchen angegeben.

Da die Lichtreflexion nur an der Endfläche des Lichtleiters, d.h. z.B. einer Glas- oder Kunststoffaser durch die Flüssigkeit beeinflußt wird, ist die effektive Sondendicke auf die Lichtwellenlänge und der Sondendurchmesser auf die optische Faserdicke begrenzt.

Das Meßverfahren kann auch bei trüben Flüssigkeiten angewendet werden, wenn die unmittelbare Sondenumgebung durch eine optisch einwandfreie z.B. in einem Ballon befindliche Flüssigkeit gebildet wird. Diese kann auch durch einen geeigneten optischen, störungsfreien, gummielastischen oder festen Körper wie z.B. aus Plexiglas oder Polyurethan ersetzt werden. Hierdurch wird es möglich die Sonde auch im medizinischen Bereich z.B. für Stoßwellenmessungen im Körperinneren per Katheter oder in Verbindung mit endoskopischen Methoden einzusetzen.

Zur Optimierung der Sondenempfindlichkeit und Linearität der Anzeige ist es günstig, den Brechungsindex der Flüssigkeit oder des Körpers hoher optischer Qualität, d.h. optisch klar, oder auch den Brechungsindex des Lichtleiters jeweils auszuwählen.

Ferner kann durch geeignete Wahl der Lichtfrequenz z.B. auf der Flanke eines molekularen Schwingungsübergangs oder auch eines elektronischen Übergangs eine besonders starke Druckabhängigkeit des Brechungsindexes durch Druckverschiebung des entsprechenden optischen Übergangs zu erheblichen Empfindlichkeitssteigerungen führen. Im sichtbaren und ultravioletten Spektralbereich können hierbei der Flüssigkeit zugesetzte Farbstoffmoleküle günstige Eigenschaften besitzen. Entsprechende Möglichkeiten bestehen auch für eine Dotierung des Lichtwellenleiters (Glasfaser oder Kunststoff) mit Atomen oder Molekülen, die eine starke Druckabhängigkeit des Brechungsindexes und damit der Lichtreflexion aufweisen.

Auch der Grenzwinkel der Totalreflexion wird durch die druckinduzierte Brechungsindexänderung beeinflußt. Bei optischem Strahlengang im Lichtleiter mit Bevorzugung des Totalreflexionsgrenzwinkels kann dieser Einfluß auf die gesamte reflektierte Lichtintensität überwiegen.

Die Art der am Lichtleiterende für die Reflexion wesentlichen Fläche kann auf verschiedene Weise gewählt werden. Einmal ist eine ebene polierte Endfläche geeignet. Ebene Flächen guter optischer Qualität können jedoch auch leicht durch Anritzen und Brechen hergestellt werden. Ebenso erhält man durch eine Halbkugel- oder Kugelform des Lichtleiterendes, z.B. durch Anschmelzen, ideale Oberflächeneigenschaften, wobei für alle Lichtwinkel des Wellenleiters ein senkrechter Grenzflächeneinfall erreicht werden kann und damit auch eine für Eichzwecke wichtige einfache rechnerische Behandlung ermöglicht wird.

Eine möglichst einfache, leistungsfähige, optische Meßanordnung zur Erfassung des Reflexionssignals und dessen Umsetzung in ein Drucksignal besteht aus einem Dauerstrich-Laser hoher Leistung (bis zu 1 Watt), einer Einkopplung der Laserenergie über einen optischen Koppler und einem Fotodetektor zur Messung der reflektierten Lichtintensität mit nachfolgendem Verstärker. Um Amplitudenschwankungen des primären Laserlichts berücksichtigen zu können, wird ein direkt ausgekoppelter Lasersignalanteil mit dem Sondensignal, z.B. durch Subtraktion, verglichen. Dieser Lasersignalanteil kann außerdem zur Konstantregelung der Laserintensität herangezogen werden.

Insgesamt zeichnet sich die Erfindung gegenüber dem Stand der Technik durch folgende Vorteile aus:
Als optische Wellenleiter können gängige Glasfasertypen oder Polymerfasern verwendet werden. Die allein empfindliche Sondenfläche kann durch Brechen oder Politur hergestellt werden. Die effektiven Sondendimensionen können kleiner als 0,1 mm gewählt werden. Das empfindliche Volumen wird durch den Faserdurchmesser und die halbe Lichtwellenlänge gebildet. Bei senkrechter Druckwelleninzidenz in Wasser entspricht dies einer maximalen Bandbreite von 30 GHz. Durchmesser und Länge des Lichtleiters besitzen keinen direkten Einfluß auf die Empfindlichkeit. Daher sind geringe Durchmesser zur Erzielung großer Bandbreite auch bei seitlicher Inzidenz, d.h. einer idealen Kugelcharakteristik sowie große Längen, z.B. für medizinische Anwendungen, im Gegensatz zu piezoelektrischen Sondenhydrophonen unproblematisch. Die Empfindlichkeit wird demgegenüber durch das Photonenrauschen begrenzt, das je nach Eingangslichtleistung jedoch genügend stark reduziert werden kann. Der Einfluß von sonstigen Rauschanteilen der Eingangslichtquelle kann entweder durch Vergleichsmethoden oder durch Regelung herabgesetzt werden. Elektrische Störungen, z.B. durch Funkenentladung bei der Stoßwellenerzeugung, werden wegen der idealen Isolation der Glasfaser optimal abgeschirmt. Ebenso ist gerade für Stoßwellenmessungen die Lebensdauer des Glasfaserhydrophons wegen des wesentlich einfacheren Aufbaus gegenüber piezoelektrischen Hydrophonen erheblich höher. Auch kann bei eventueller Beschädigung des Glasfaserendes leicht eine neue Glasfaser-Reflexionsfläche hergestellt werden. Insgesamt besitzt das optische Reflexions-Sondenhydrophon somit eine Reihe von wichtigen Vorteilen gegenüber bekannten piezoelektrischen Sondenhydrophonen.

Die Erfindung ist anhand von Ausführungsbeispielen in den Zeichnungen 1 bis 3 dargestellt und nachfolgend erläutert.

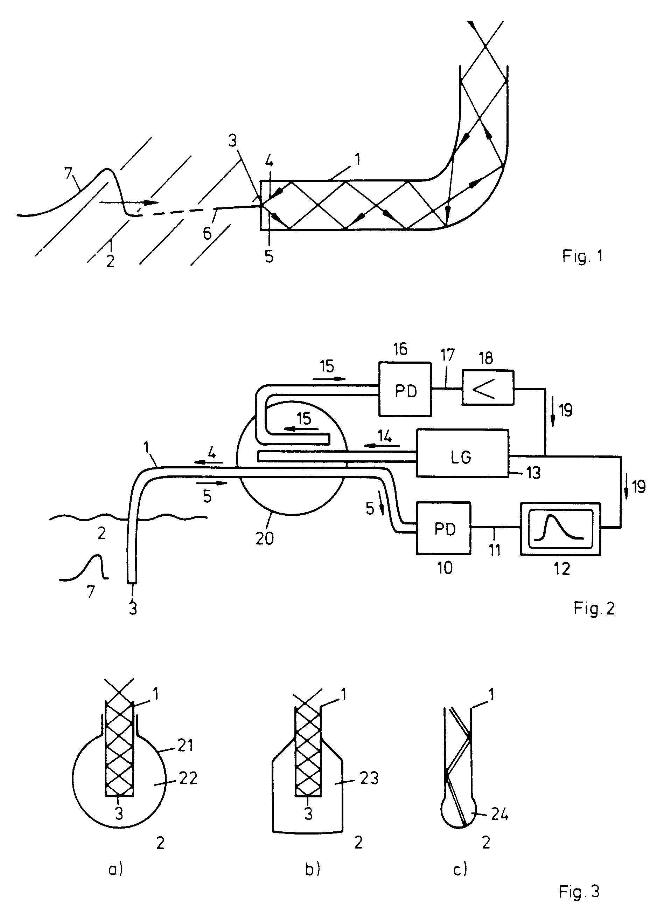

Fig. 1 zeigt die in die Flüssigkeit 2 eintauchende Glasfaser 1 mit der reflektierenden Endfläche 3. Das eintretende Licht 4 wird im Lichtleiter bis zur Grenzfläche 3 geführt, dort z.T. reflektiert 5 und z.T. in die Flüssigkeit durchgelassen 6. Unter dem Einfluß einer Druckwelle 7 wird die Flüssigkeit 2 vor der Grenzfläche 3 verdichtet, so daß die kurzzeitige Erhöhung des Brechungsindexes in der Flüssigkeit zu einer Absenkung der Intensität des reflektierten Lichtes 5 führt. Das reflektierte Licht 5 wird von einem Fotodetektor 10 (Fig.2) in ein elektrisches Signal 11 umgewandelt und mittels Oszilloskop 12 registriert. Fig. 3 a, b, c zeigt unterschiedliche Ausführungsformen des Lichtleiterendes 3 in der Flüssigkeit 2. Bei Fig. 3 a umschließt ein Ballon 21, der mit einer optisch hochwertigen Flüssigkeit 22 gefüllt ist, das Lichtleiterende 3. Diese Ausführungsform empfiehlt sich bei lichtundurchlässigem oder trübem akustischem Medium 2. In diesem Fall ist alternativ die Ausführungsform in Fig 3 b zu wählen, bei der ein optisch klarer z.B. gummielastischer Körper oder ein anderer Kunststoffkörper 23 das Lichtleiterende 3 umgibt. In Fig. 3 c besitzt das Ende 24 des Lichtleiters 1 Kugelform. Hierdurch überwiegt an der reflektierenden Fläche der senkrechte Lichteinfall. Auf der Seite der Lichtsignalerzeugung und Verarbeitung wird im Optokoppler 20 das Licht 14 des Lichtgenerators 13 (Laser) in das einfallende Licht des Meßzweigs 4 und einen Lichtanteil im Vergleichszweig 15 aufgeteilt. Die Signalerzeugung aus dem Meßlicht 4 bis zur Darstellung auf dem Oszilloskop 12 wurde bereits beschrieben. Demgegenüber wird das Vergleichslicht 15 über eine Fotozelle 16 in ein elektrisches Vergleichssinal 17 und nach Verstärkung über Verstärker 18 als Steuer und Regelsignal 19 zur Laserrauschreduktion benutzt. Das Vergleichssignal 19 wird am Oszillographen 12 vom Meßsignal 11 bei gleicher Ruhepegeleinstellung subtrahiert. Gleichzeitig erfolgt eine Konstantregelung des Lasersignals 14 mit Hilfe des Vergleichssignals 19 als Regelgröße.

## Patentansprüche

1. Sondenhydrophon zur Messung von Druckamplituden in einem flüssigen Medium (2), gekennzeichnet durch
- einen Lichtleiter in Form eines optischen Wellenleiters (1), der mit einem Ende in das flüssige Medium (2) eingetaucht ist,
- eine Lichtquelle (13), deren Licht in den Lichtleiter (1) eingekoppelt wird, und
- eine zeitlich hochauflösende optische Reflexionsmeßanordnung (10, 11, 12) am anderen Ende des Lichtleiters (1), welche die Änderung des Lichtreflexionsgrades an der Grenzfläche (3) von eingetauchtem Lichtleiterende und flüssigem Medium erfaßt und daraus die zugehörigen Druckamplituden mit hoher zeitlicher Auflösung mit einer Bandbreite von bis zu 30 GHz ermittelt.

2. Sondenhydrophon nach Anspruch 1, dadurch gekennzeichnet, daß das sich in der Flüssigkeit befindende Lichtleiterende (3) von einem mit einer optisch klaren Flüssigkeit (22) gefüllten Ballon (21) umgeben ist.

3. Sondenhydrophon nach Anspruch 1, dadurch gekennzeichnet, daß das sich in der Flüssigkeit befindende Lichtleiterende (3) von einem optisch klaren Körper (23) aus einem z.B. gummielastischen festen Medium umgeben ist.

4. Sondenhydrophon nach Anspruch 2 oder 3, dadurch gekennzeichnet, daß die optisch klare Flüssigkeit (22) oder der optisch klare Körper (23) aus einer Gruppe von Flüssigkeiten und Körpern mit unterschiedlichen Brechungsindizes auswählbar ist.

5. Sondenhydrophon nach einem der Ansprüche 1 bis 4, dadurch gekennzeichnet, daß je nach dem umgebenden flüssigen Medium (2, 22) oder Körper (23) ein Lichtleiter (1) mit jeweils geeignetem Brechungsindex vorgesehen ist.

6. Sondenhydrophon nach einem der Ansprüche 1 bis 5, dadurch gekennzeichnet, daß die Lichtfrequenz auf einen Molekülübergang oder eine Molekülrelaxation der umgebenden Flüssigkeit (2, 22) oder des Körpers (23) oder des Lichtleiters (1) abgestimmt ist.

7. Sondenhydrophon nach einem der Ansprüche 1 bis 6, dadurch gekennzeichnet, daß die Lichtquelle (13) aus einer Gruppe von Lichtquellen ausgewählt ist, die Licht mit einer jeweils unterschiedlichen Frequenz vom UV-Bereich bis zum Ferninfrarot-Bereich emittieren.

8. Sondenhydrophon nach einem der Ansprüche 1 bis 7, dadurch gekennzeichnet, daß zusätzlich zur Änderung des Lichtreflexionsgrades eine Änderung des Grenzwinkels der Totalreflexion für die Druckamplitudenmessung ausgenutzt wird.

9. Sondenhydrophon nach einem der Ansprüche 1 bis 8, dadurch gekennzeichnet, daß das Ende (3) des Lichtleiters (1) poliert ist.

10. Sondenhydrophon nach einem der Ansprüche 1 bis 8, dadurch gekennzeichnet, daß das Ende (3) des Lichtleiters (1) eine ebene Bruch- oder Spaltfläche ist.

11. Sondenhydrophon nach einem der Ansprüche 1 bis 8, dadurch gekennzeichnet, daß das Ende des Lichtleiters (1) kugelförmig ist.

12. Sondenhydrophon nach einem der Ansprüche 1 bis 11, dadurch gekennzeichnet, daß zur Einkopplung des einfallenden Lichtes der Lichtquelle (13) ein optischer Koppler (20) vorgesehen ist, an dessen Ende ein Photodetektor (10) mit nachfolgendem Verstärker (18) zur Erzeugung eines Vergleichssignals (19) für die Reflexionsmeßanordnung (10, 11, 12) zur Erfassung der Änderung des Lichtreflexionsgrades angeordnet ist.

13. Sondenhydrophon nach Anspruch 12, dadurch gekennzeichnet, daß von der Lichtquelle (13) ein Teil des Lichtes direkt abgezweigt (15) und dem Photodetektor (16) mit Verstärker (18) zugeführt ist, dessen Ausgangssignal (19) mit dem Signal (11) des reflektierten Lichtes (5) verglichen wird.

14. Sondenhydrophon nach Anspruch 13, dadurch gekennzeichnet, daß das Ausgangssignal (19) des Verstärkers (18) zusätzlich der Lichtquelle (13) zur Konstantregelung der von dieser erzeugten Lichtintensität zugeführt ist.

## Claims

1. A probe hydrophone for measuring amplitudes of pressure in a liquid medium (2) characterised by
- a light guide in the form of an optical wave guide (1), one end of which is immersed in the liquid medium (2),
- a light source (13), the light from which is fed into the light conductor (1), and
- a time-based high-resolution optical reflection measuring arrangement (10, 11, 12) at the other end of the light conductor (1) which detects the fluctuation in degree of light reflection at the interface (3) of immersed light conductor end and liquid medium and ascertains therefrom the associated pressure amplitudes with a high time-based resolution with a band width of up to 30 GHz.

2. A probe hydrophone according to claim 1, characterised in that the end (3) of the light conductor which is in the liquid is enclosed by a balloon (21) filled with an optically clear liquid (22).

3. A probe hydrophone according to claim 1, characterised in that the end (3) of the light conductor which is in the liquid is enclosed by an optically clear body (23) consisting of a for instance rubber-elastic solid medium.

4. A probe hydrophone according to claim 2 or 3, characterised in that the optically clear liquid (22) or the optically clear body (23) can be chosen from a group of liquids and bodies having different refraction indices.

5. A probe hydrophone according to one of claims 1 to 4, characterised in that according to the surrounding liquid medium (2, 22) or body (23), so a light conductor (1) of an appropriately suitable refraction index is provided.

6. A probe hydrophone according to one of claims 1 to 5, characterised in that the light frequency is attuned to a molecular transfer or a molecular relaxation in the surrounding liquid (2, 22) o9r body (23) or light conductor (1).

7. A probe hydrophone according to one of claims 1 to 6, characterised in that the light source (13) is chosen from a group of light sources emitting light with a respectively different frequency from the ultra-violet range to the remote infra-red range.

8. A probe hydrophone according to one of claims 1 to 7, characterised in that in addition to the fluctuation in the degree of light reflection, a variation in the limit angle of total reflection is used for measuring pressure amplitudes.

9. A probe hydrophone according to one of claims 1 to 8, characterised in that the end (3) of the light conductor (1) is polished.

10. A probe hydrophone according to one of claims 1 to 8, characterised in that the end (3) of the light conductor (1) is a plane fractured or split surface.

11. A probe hydrophone according to one of claims 1 to 8, characterised in that the end of the light conductor (1) is spherical.

12. A probe hydrophone according to one of claims 1 to 11, characterised in that for feeding in the incident light from the light source (13) an optical coupler (20) is provided at the end of which there is a photodetector (10) with, on the downstream side, an amplifier (18) for generating a comparison signal (19) for the reflection measuring arrangement (10, 11, 12) for detecting the fluctuation in the degree of light reflection.

13. A probe hydrophone according to claim 12, characterised in that part of the light from the light source (13) is directly branched off (15) and is fed to the photodetector (16) with amplifier (18), the output signal (19) from which is compared with the signal (11) for the reflected light (5).

14. A probe hydrophone according to claim 13, characterised in that the output signal (19) from the amplifier (18) is additionally fed to the light source (13) to maintain constant the light intensity generated by said light source.

## Revendications

1. Hydrophone de sondage, pour mesurer des amplitudes de pression dans un milieu liquide (2), caractérisé en ce qu'il contient :
- une fibre optique sous forme d'un guide d'ondes optiques (1), qui par une extrémité est plongée dans le milieu liquide (2),
- une source de lumière (13), dont la lumière est couplée dans la fibre optique (1), et
- un dispositif de mesure de la réflexion optique, à grande résolution dans le temps (10, 11, 12) à l'autre extrémité de la fibre optique (1), qui capte la variation du facteur de réflexion lumineuse au niveau de l'interface (3) entre l'extrémité immergée de la fibre optique et le milieu liquide, et calcule à partir de ce facteur de réflexion les amplitudes de pression correspondantes, avec une grande résolution dans le temps et une largeur de bande allant jusqu'à 30 GHz.

2. Hydrophone de sondage selon la revendication 1, caractérisé en ce que l'extrémité (3) de la fibre optique se trouvant dans le liquide est entourée d'un ballon (21), rempli d'un liquide (22) optiquement transparent.

3. Hydrophone de sondage selon la revendication 1, caractérisé en ce que l'extrémité (3) de la fibre optique se trouvant dans le liquide est entourée d'un corps optiquement transparent (23), par exemple en un milieu solide ayant l'élasticité du caoutchouc.

4. Hydrophone de sondage selon la revendication 2 ou 3, caractérisé en ce que le liquide optiquement transparent (22) ou le corps optiquement transparent (23) peut être choisi parmi un ensemble de liquides et de corps ayant différents indices de réfraction.

5. Hydrophone de sondage selon l'une des revendications 1 à 4, caractérisé en ce que, selon le milieu liquide (2, 22) ou le corps (23) qui entoure son extrémité, on prévoit une libre optique (1) ayant un indice de réfraction approprié.

6. Hydrophone de sondage selon l'une des revendications 1 à 5, caractérisé en ce que la fréquence lumineuse est adaptée à une transition moléculaire ou à une relaxation moléculaire du liquide (2, 22) ou du corps (23) entourant l'extrémité de la libre optique, ou encore de la fibre optique (1).

7. Hydrophone de sondage selon l'une des revendications 1 à 6, caractérisé en ce que la source lumineuse (13) est choisie parmi un ensemble de sources lumineuses qui émettent une lumière ayant une fréquence toujours différente, allant de la plage des UV à celle des infrarouges lointains.

8. Hydrophone de sondage selon l'une des revendications 1 à 7, caractérisé en ce qu'on utilise pour mesurer les amplitudes de pression, en plus d'une variation du facteur de réflexion lumineuse, une variation de l'angle limite de la réflexion totale.

9. Hydrophone de sondage selon l'une des revendications 1 à 8, caractérisé en ce que l'extrémité (3) de la fibre optique (1) est polie.

10. Hydrophone de sondage selon l'une des revendications 1 à 8, caractérisé en ce que l'extrémité (3) de la libre optique (1) présente une surface de rupture ou de clivage plane.

11. Hydrophone de sondage selon l'une des revendications 1 à 8, caractérisé en ce que l'extrémité de la fibre optique (1) est sphérique.

12. Hydrophone de sondage selon l'une des revendications 1 à 11, caractérisé en ce que, pour coupler la lumière incidente de la source lumineuse (13), on prévoit un coupleur optique (20) en l'extrémité duquel est disposé un photodétecteur (10), suivi d'un amplificateur (18) destiné à produire un signal de comparaison (19) pour le dispositif de mesure de la réflexion (10, 11, 12), dans le but de détecter la variation du facteur de réflexion lumineuse.

13. Hydrophone de sondage selon la revendication 12, caractérisé en ce qu'on dérive directement (15) une partie de la lumière provenant de la source lumineuse (13), et on l'envoie au photodétecteur (16) muni de l'amplificateur (18), dont le signal de sortie (19) est comparé au signal (11) de la lumière réfléchie (5).

14. Hydrophone de sondage selon la revendication 13, caractérisé en ce que le signal de sortie (19) de l'amplificateur (18) est en outre envoyé à la source lumineuse (19) pour réguler, en maintenant constante, l'intensité lumineuse produite par cette dernière.
